# EUROPEAN PATENT APPLICATION

(11) **EP 0 895 778 A2**
(43) Date of publication of application: **10.02.1999**
(21) Application number: 98306152.4
(22) Date of filing: 31.07.1998
(51) Int. Cl.: A61K 7/48, A61K 7/06

(54) **Cosmetic composition**

(30) Priority: 04.08.1997 JP 209478/97; 26.12.1997 JP 360289/97
(71) Applicant: Ajinomoto Co., Inc., Tokyo 104-0031 (JP)
(72) Inventor: Ogawa, Masumi, c/o Ajinomoto Co., Inc., Kawasaki-shi, Kanagawa-ken 210-0801 (JP); Tabohashi, Tatsuru, c/o Ajinomoto Co., Inc., Kawasaki-shi, Kanagawa-ken 210-0801 (JP)
(74) Representative: Nicholls, Kathryn Margaret

(57) **Abstract**

Herein is disclosed a cosmetic composition containing, as the active ingredient, at least one member selected from the group consisting of basic amino acid derivatives of formula (1) and their salts (Component A), and at least one member selected from the group consisting of natural, mineral and synthetic oily materials for cosmetics (Component B).

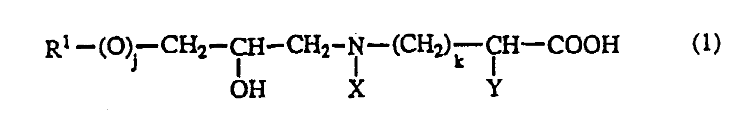

## Description

The present invention relates to a cosmetic composition.

A cosmetic composition according to the present invention can exhibit, when applied to the hair, an excellent hair-conditioning effect for smooth combing, sleeking and wet feeling with no stickiness, and can exhibit, when applied to the skin, good affinity for, and good moisturizing effect on, the skin with no stickiness.

Some hair cosmetic compositions including shampoo and rinse are often problematic in that the oily component of the hair having been shampooed therewith is so much removed that the shampooed hair gets a dry and rough feel and is difficult to smoothly comb. As one means of solving this problem, some oily materials for cosmetic use capable of making the hair have an oily and soft feel while preventing the hair from being too much dried may be added to such compositions. Adding such oily materials will prevent the shampooed hair from getting a dry and rough feel, while making the hair well combable.

However, oily materials for cosmetic use are often defective in that hair cosmetics comprising them will give an oily and sticky feel to the hair. In addition, their effect of conditioning the hair for smooth combing, sleeking and wet feeling is not satisfactory.

Alkyl quaternary ammonium salts such as dialkyldimethylammonium chlorides and monoalkyltrimethylammonium chlorides may be used in hair cosmetics for enhancing the hair-conditioning effect of such cosmetics. However, those alkyl quaternary ammonium salts will much irritate the skin and ocular mucous membranes, and are poorly biodegradable, and they can not remove the sticky feeling to be derived from oily materials in cosmetics.

As other conditioning agents than alkyl quaternary ammonium salts are known alkylamide amine salts such as stearic acid diethylaminoethylamide, etc., and cocoylarginine ethyl ester pyrrolidone-carboxylates, etc. However, they are not comparable to alkyl quaternary ammonium salts in point of their conditioning effect, though being poorly irritable and highly biodegradable compared therewith.

On the other hand, oily materials for cosmetic use are very often used in cosmetics such as lotion, essence, cream, lipstick, foundation, etc., essentially as the binder for cosmetic ingredients, or as the emollient or the preparation shape retainer. However, they are often defective, as causing poorer affinity for the skin or stickiness.

Given that situation, it is desired to develop cosmetics having the advantages of good characteristics of oily materials for cosmetics and wholly or partially free from those disadvantages noted above of the oily materials.

Embodiments of the present invention can provide a cosmetic composition which exhibits, when applied to the hair, an excellent hair-conditioning effect for smooth combing, sleeking and wet feeling with no stickiness, and exhibits, when applied to the skin, good affinity for, and good moisturizing effect on, the skin with no stickiness.

Considering the current situation noted above, the present inventors have assiduously studied, and, as a result, have found that the above-mentioned object can be attained by a combination of a natural, mineral or synthetic oily material for cosmetic use (Component B) and a basic amino acid derivative or its salt (Component A) which can be produced by reaction of a specific compound, i.e., a glycidyl ether or an epoxyalkane, with a basic amino acid such as arginine, lysine or the like. On the basis of these findings, they have completed the present invention.

Specifically, the invention relates to a cosmetic composition comprising, as the active ingredient, at least one member selected from the group consisting of basic amino acid derivatives of the following general formula (1) and their salts (Component A) and at least one member selected from the group consisting of natural, mineral and synthetic oily materials for cosmetic use (Component B).

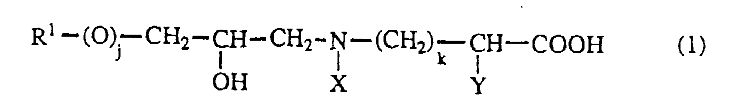

In Formula (1), R¹ represents a straight-chain or branched-chain alkyl or alkenyl group having from 8 to 22 carbon atoms; j represents an integer of 0 or 1; X represents a hydrogen atom or a substituent of the following general formula (2); k represents an integer of from 0 to 5; and Y represents a substituent of the following general formula (3) when k = 0, while Y represents an amino group when k = an integer of from 1 to 5.

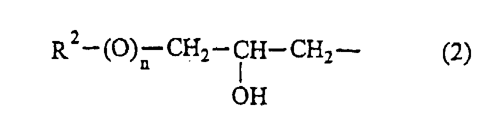

In Formula (2), R² represents a straight-chain or branched-chain alkyl or alkenyl group having from 8 to 22 carbon atoms, which may be the same as, or different from, R¹; and n represents an integer of 0 or 1.

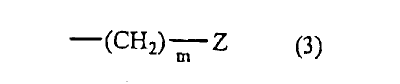

In Formula (3), m represents an integer of from 1 to 5; and Z represents a substituent of any of the following (I) to (IV).

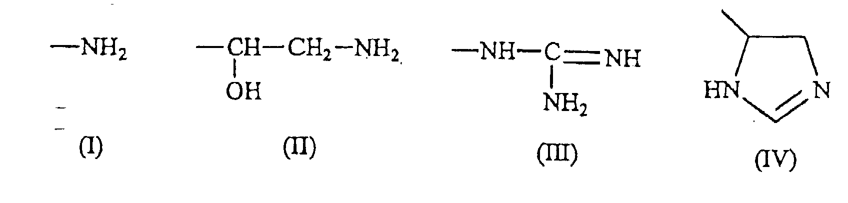

Now, embodiments of the invention will be described in detail hereinunder.

The basic amino acid derivative of Formula (1) as the first indispensable ingredient (Component A) of the cosmetic composition of the present invention can be easily produced by making a basic amino acid react with a glycidyl ether of the following general formula (4) or an epoxyalkane of the following general formula (5), under an alkaline condition in an organic solvent such as a lower alcohol, a polyhydric alcohol or the like or in a mixed solvent of such an organic solvent and water. For example, the reaction of an amino acid with an epoxyalkane is described in Japanese Patent Application Laid-Open (JP-A) Sho 48-22417. 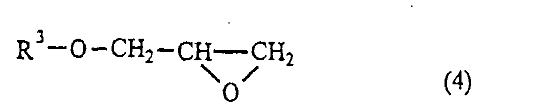 wherein R³ represents a straight-chain or branched-chain alkyl or alkenyl group having from 8 to 22 carbon atoms. 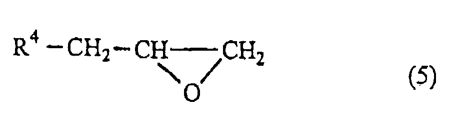 wherein R⁴ represents a straight-chain or branched-chain alkyl or alkenyl group having from 8 to 22 carbon atoms.

Of the basic amino acid derivatives of Formula (1) are preferred those produced by making a glycidyl ether react with a basic amino acid to those produced by making an epoxyalkane react with a basic amino acid. Amino acid derivatives produced by making an acidic or neutral amino acid react with a glycidyl ether or epoxyalkane do not exhibit such conditioning effect as provided by the present invention.

The glycidyl ethers of Formula (4) can be produced, for example, by making a saturated or unsaturated, natural or synthetic higher alcohol react with epichlorohydrin. Specific examples of the glycidyl ethers include decyl glycidyl ether, dodecyl glycidyl ether, tetradecyl glycidyl ether, stearyl glycidyl ether, etc. These may be used singly or as a mixture of two or more thereof in any optional ratio. Commercially available glycidyl ethers include "Epiol L-41" (decyl glycidyl ether) and "Epiol SK" (stearyl glycidyl ether) ex NOF Corporation; "Heloxy 8" (mixture of dodecyl glycidyl ether and tetradecyl glycidyl ether) ex ACI Japan, Ltd.; "Denacol EX-192" (mixture of dodecyl glycidyl ether and tetradecyl glycidyl ether) ex Nagase Chemicals, Ltd.; "SY-25L" (mixture of decyl glycidyl ether and dodecyl glycidyl ether) ex Sakamoto Yakuhin Kogyo Co., Ltd., etc.

As examples of the epoxyalkanes of Formula (5) are mentioned commercial products of "AOEX" series ex Daisel Chemical Industries, Ltd.

Any natural or synthetic basic amino acids are employable herein, which may include, for example, arginine, lysine, ornithine, histidine, hydroxylysine, a,y-diaminobutyric acid, etc. Preferred are arginine and lysine; and more preferred is arginine. Any of D-, L- and DL-forms of basic amino acids are acceptable.

In case of lysine and ornithine for example, out of basic amino acids, it is believed that they will bond to a glycidyl ether or epoxyalkane predominantly at the E-amino group and δ-amino group, respectively. However, they may bond thereto at the α-amino group. Furthermore, two molecules of a glycidyl ether or epoxyalkane may bond to any single one of the ε-amino group and the δ-amino group, or the α-amino group of the basic amino acid. One molecule of a glycidyl ether or epoxyalkane may bond to the ε-amino group (δ-amino group in case of ornithine) and another molecule, to the α-amino group, totaling two molecules; or three or four molecules thereof may bond to one molecule of the basic amino acid. Of those, however, are particularly preferred adducts composed of one molecule of a basic amino acid and one molecule of a glycidyl ether or epoxyalkane.

In the reaction of a glycidyl ether or epoxyalkane with a basic amino acid, it is desirable that the amino acid is used in the form of its alkali metal salt, or the reaction is effected under an alkaline condition, whereby the reactivity is increased while the side reaction is being suppressed. However, these conditions are not always required of arginine.

As the reaction solvent are generally used one or more organic solvents such as lower alcohols, polyhydric alcohols, etc. Examples of the lower alcohols include methyl alcohol, ethyl alcohol, n-propyl alcohol, isopropyl alcohol, n-butyl alcohol, etc.; and examples of the polyhydric alcohols include glycerin, 1,3-butylene glycol, propylene glycol, dipropylene glycol, isoprene glycol, etc. These organic solvents may be used singly or in the form of a mixture of two or more thereof in any desired ratio. Needless-to-say, mixtures of different types of organic solvents such as a higher alcohol combined with a polyhydric alcohol, etc, are employable. In order to increase the solubility of basic amino acids therein, any one of those organic solvents may be more preferably used together with water. The mixing ratio of the organic solvent such as a lower alcohol, a polyhydric alcohol or the like to water varies depending on the type of the amino acids and on the type of the glycidyl ethers or epoxyalkanes to react therewith, but the ratio of the organic solvent to water generally falls between 100 : 0 and 10 : 90, preferably between 1 : 1 and 9 : 1. If the ratio of the organic solvent is too low, the solubility of the glycidyl ether or epoxyalkane in the solvent will be low whereby the reaction speed will be much lowered.

The reaction temperature varies, depending on the type and the composition of the reaction solvent used. For example, where isopropyl alcohol is used, the reaction temperature may fall generally between 70 and 100°C, preferably between 80 and 95°C; and where ethyl alcohol is used, it may fall generally between 60 and 78°C, preferably between 70 and 78°C. The reaction is desirably effected under reflux of the reaction system. Regarding the way of adding a glycidyl ether or epoxyalkane to a basic amino acid, it may be added thereto all at a time prior to heating the reaction system, or may be added intermittently in portions or continuously (continuous dropwise addition) after the start of the heating. In order to inhibit the formation of by- products, however, the continuous dropwise addition is preferred after the start of the heating.

In general, the product produced by the reaction of a glycidyl ether or epoxyalkane with a basic amino acid is not a single compound but is a mixture of an adduct of one molecule of the basic amino acid and one molecule of the glycidyl ether or epoxyalkane, and adducts of one molecule of the former and two or more molecules of the latter. The mixture may be purified and separated into the single compounds, for example, through chromatography or the like, and one or more of the thus-isolated single compounds may be used in producing the cosmetic composition of the present invention; or alternatively, the mixture per se may be directly used in the composition. The non-reacted basic amino acid may remain in the mixture.

In the basic amino acid derivative for use in the invention, which is composed of one molecule of a basic amino acid and plural molecules of a glycidyl ether or epoxyalkane added thereto, the plural glycidyl ether or epoxyalkane molecules added may be the same or different in their chain length. Where a mixture of plural glycidyl ethers or epoxyalkanes each having a different chain length is made to react with a basic amino acid, the resulting product is generally a mixture of basic amino acid derivatives of which the plural glycidyl ether or epoxyalkane moieties are the same or different in their chain length. The mixture may be purified and separated into the single compounds in the manner mentioned above, or may be directly used as it is.

Where a mixture of a glycidyl ether and an epoxyalkane is made to react with a basic amino acid, the resulting product generally contains a basic amino acid derivative of such a type that both the glycidyl ether and the epoxyalkane are added to one molecule of the basic amino acid. The product of this type may also be purified and separated into the single compounds in the manner mentioned above, but may be directly used as it is.

The reaction mixture as obtained by making a glycidyl ether or epoxyalkane react with a basic amino acid may be directly used as it is, if possible, without purifying it, but if desired, it may be purified to such a degree that the reaction solvent is evaporated out, prior to being used for producing the cosmetic composition of the present invention. Where ethyl alcohol, 1,3-butylene glycol, propylene glycol or the like, for example, which is generally used in producing cosmetics with no problems, is used as the reaction solvent in producing the basic amino acid derivative for use in the present invention, the reaction mixture may still contain such a solvent remaining insofar as it does not interfere with the effect of the present invention.

Examples of the salts of the basic amino acid derivative of Formula (1) include inorganic acid salts such as hydrochlorides, hydrobromides, sulfates, phosphates, nitrates, carbonates, etc.; and organic acid salts such as acetates, citrates, lactates, glycolates, malates, p-toluenesulfonates, tartrates, glutamates and salts of their derivatives, aspartates and salts of their derivatives, pyrrolidone-carboxylates, etc. Any optical isomers of those salts are acceptable. Especially preferred salts are hydrochlorides, hydrobromides, pyrrolidone-carboxylates, citrates, lactates, glycolates, glutamates, aspartates, etc.

Next, natural, mineral and synthetic oily materials for cosmetic use as the second indispensable ingredient (Component B) of the cosmetic composition of the present invention will be mentioned hereinunder with reference to their specific examples.

The natural oily materials for cosmetic use include bees wax, bleached bees wax, candelilla wax, carnauba wax, insect wax, haze wax, rice bran wax, bleached montan wax, lanolin, ceresine, squalane, pristane, turpentine oil, eucalyptus oil, terpineol, eucalyptol, olive oil, tsubaki oil (camellia oil), tea seed oil, sasanqua oil, safflower oil, sunflower oil, soybean oil, cotton seed oil, sesame oil, corn oil, peanut oil, rapeseed oil, rice bran oil,rice germ oil, Job's tears oil, grape seed oil, almond oil, jojoba oil, avocado oil, carrot oil, macadamia nut oil, hazel nut oil, meadow foam oil, shea butter, evening primrose oil, cacao butter, castor oil, hardened castor oil, linseed oil, coconut oil, palm, oil, palm kernel oil, beef tallow, horse oil, mink oil, cod liver oil, shark liver oil, orange raffia oil, milk butter, egg yolk oil, egg yolk fatty oil, powdery fatty oil, clove oil, rose hip oil, lavender oil, Roman chamomile oil, rosemary oil, etc. Partially chemically modified, natural oily materials for cosmetic use are also employable, which include, for example, partially-hydrolyzed jojoba oil, hydroxystearic acid-hardened castor oil, castor oil acetate, partially-hydrogenated horse oil, pure lanolin obtained through adsorption purification, liquid lanolin, reduced lanolin, hard lanolin, hard lanolin acetate, liquid lanolin acetate, etc. Of those are much used bees wax, candelilla wax, lanolin, soybean oil, jojoba oil, shea butter, hardened castor oil, and modified lanolins.

The mineral oily materials for cosmetic use include liquid paraffin, light paraffin, heavy paraffin, volatile paraffin, liquefied petroleum gas, vaseline, microcrystalline wax, etc. Of these are much used liquid paraffin, vaseline and microcrystalline wax.

The synthetic oily materials for cosmetic use include higher fatty acid esters such as cetyllanolyl acetate, triacetylglyceryl, eicosyl propionate, lauryl lactate, myristyl lactate, cetyl lactate, octyldodecyl lactate, diisostearyl malate, polypropylene glycol succinate oligoester, 2-ethylhexyl succinate, stearyl heptanoate, diisopropyl adipate, dibutyl adipate, dioctyl adipate, di-2-ethylhexyl adipate, di-2-heptylundecyl adipate, cetyl caprylate, glyceryl tricaprate, cetyl 2-ethylhexanoate, cetostearyl 2-ethylhexanoate, glyceryl tri-2-ethylhexanoate, pentaerythritol tetra-2-ethylhexanoate, isocetyl octanoate, isostearyl octanoate, ethylene glycol dioctanoate, neopentyl glycol dioctanoate, trimethylolpropane trioctanoate, hexyldecyl dimethyloctanoate, octyldodecyl dimethyloctanoate, isononyl isononanoate, isodecyl isononanoate, isotridecyl isononanoate, isotridecyl dinonanoate, propylene glycol dinonanoate, octyl pelargonate, octyl isopelargonate, glycerin tri(caprylcaproate), neopentyl glycol dicaprate, diisopropyl sebacate, hexyl laurate, isostearyl laurate, glyceryl trilaurate, glyceryl tri-coconut oil fatty acid ester, isopropyl myristate, butyl myristate, myristyl myristate, cetyl myristate, isocetyl myristate, isostearyl myristate, octyldodecyl myristate, glyceryl trimyristate, pentaerythritol tetramyristate, isopropyl palmitate, octyl palmitate, cetyl palmitate, isocetyl palmitate, 2-ethylhexyl palmitate, octyl isopalmitate, glyceryl triisomyristate, ethyl stearate, octyl stearate, isocetyl stearate, stearyl stearate, hardened castor oil stearate, glyceryl tristearate, butyl stearate, isopropyl isostearate, butyl isostearate, hexyl isostearate, isocetyl isostearate, isostearyl isostearate, hardened castor oil isostearate, octyldodecyl isostearate, butyl isostearate, polyglyceryl monoisostearate, glyceryl tri(isostearate), diglyceryl tri(isostearate), trimethylolpropane tri(isostearate), polyglyceryl tetra(isostearate), polyglyceryl tetra(isostearate), 2-ethylhexyl hydroxystearate, ethyl oleate, decyl oleate, isodecyl oleate, oleyl oleate, oleyldodecyl oleate, ethylene glycol di(oleate), glyceryl tri(oleate), octyldodecyl ricinoleate, isodecyl pivalate, isostearyl pivalate, glyceryl tribehenate, octyldodecyl erucate, isopropyl lanolin fatty acid ester, octyldodecyl lanolin fatty acid ester, ethyl avocado oil fatty acid ester, ethyl mink oil fatty acid ester, dipentaerythritol fatty acid ester, dipentaerythritol hexahydroxystearate, etc.; as well as di(cholesterol, behenyl, octyldodecyl) N-lauroyl-L-glutamate, di(behenyl, octyldodecyl) N-lauroyl-L-glutamate, di(phytosteryl, 2-octyldodecyl) N-lauroyl-L-glutamate, etc. Of those, higher fatty acid esters are much used.

The amount of Component A to be in the cosmetic composition of the present invention may be suitably determined, depending on the intended product, and is not specifically defined. Preferably, however, it is from 0.01 to 10 % by weight, more preferably from 0.1 to 5 % by weight. If the amount is smaller than 0.01 % by weight, the composition could not satisfactorily exhibit the effect of the present invention. However, if the amount is larger than 10 % by weight, the composition, when applied to the hair, will make the hair roughened or poorly combable, and, when applied to the skin, it will make the skin have an unfavorable slimy feel.

The amount of Component B to be in the cosmetic composition of the present invention may be also suitably determined, depending on the intended product, and is not specifically defined. In general, it may be from 0.01 to 50 % by weight.

The cosmetic composition of the present invention has many applications to various hair cosmetics including shampoo, rinse, rinse-in-shampoo, conditioning shampoo, hair lotion, hair conditioner, hair treatment, hear cream, hair spray, hair liquid, hair wax, hair water, hair-styling gel, hair-styling preparation, perming liquid, hair color, acidic hair color, hair manicure, etc.; and to various skin cosmetics including lotion, milky lotion, face wash, makeup remover, cleansing lotion, emollient lotion, nourishing cream, emollient cream, massage cream, cleansing cream, body shampoo, hand soap, shaving lotion, sunburn lotion, deodorant powder, deodorant lotion, deodorant spray, makeup removing gel, moisture gel, moisture essence, UV-preventing essence, shaving foam, face powder, foundation, lipstick, cheek rouge, eyeliner, eye shadow, eyebrow pencil, bathing preparation, etc.

The cosmetic composition of the present invention may optionally contain any other surfactants insofar as it does not interfere with the effect of the present invention. Examples of the additional surfactants include anionic surfactants such as alkyl sulfate salts, alkyl phosphate salts, polyoxyethylene alkyl ether sulfate salts, polyoxyethylene alkylphenyl ether sulfate salts, polyoxyethylene alkylcarboxylate salts, alkylphenyl ether sulfonate salts, salts of alkylsulfosuccinic acids and their derivatives, salts of alkylsarcosines and their derivatives, N-alkyl-N-methyl-β-alanine salts, polyoxyethylene coconut oil fatty acid monoethanolamide sulfate salts, polyoxyethylene alkyl ether phosphate salts, long-chain fatty acid ethyl ester sulfonate salts, N-coconut oil fatty acid acylglutaminate salts, N-lauroylglytaminate salts, N-myristoylglutaminate salts, N-coconut oil fatty acid acylaspartate salts, N-coconut oil fatty acid acylglycine salts, N-coconut oil fatty acid acylalanine salts, and other N-acylamino acid salts and higher fatty acid salts, etc.; amphoteric surfactants such as carbobetaine-type amphoteric surfactants, amidobetaine-type amphoteric surfactants, sulfobetaine-type amphoteric surfactants, hydroxysulfobetaine-type amphoteric surfactants, amidosulfobetaine-type amphoteric surfactants, phosphobetaine-type amphoteric surfactants, imidazoline-type amphoteric surfactants; and cationic surfactants such as amidoamines, N-acylarginine ester salts, N-[3-alkyloxy-2-hydroxypropyl]-L-arginine salts, etc. Salts of the anionic surfactants include sodium, magnesium, potassium, ammonium, diethanolamine, triethanolamine, arginine and lysine salts, etc.

In addition to the above-mentioned surfactants, any other various ordinary additives may be added to the cosmetic composition of the present invention insofar as they do not interfere with the effect of the present invention. Examples of such additives include silicone compounds, polymer substances, alcohols, polyhydric alcohols, higher alcohols, extracts, amino acids, nucleic acids, vitamins, enzymes, anti-inflammatory agents, microbicides, preservatives, anti-oxidants, UV absorbents, chelating agents, sweat retardants, pigments, dyes, acid dyes, powders for cosmetic use, pH regulators, pearly additives, moisturizers, extracts, etc., which are described in various official documents such as Standards of Cosmetic Materials, Specifications for Ingredients of Different Types of Cosmetics, Specifications for Materials of Medical-use Cosmetics, the Japanese Pharmacopeia, Official Specifications for Food Additives, etc.

### EXAMPLES

Now, the invention will be described in more detail with reference to the following Examples including Production Examples, which, however, are not intended to restrict the scope of the invention.

### Production Example 1:

45.9 g (0.26 mols) of L-arginine, 29.2 g of water and 42.2 g of ethanol were put into a three-neck, round-bottomed flask, and heated under reflux at 75°C with stirring to give a dispersion. To this was dropwise added 73.6 g (0.29 mols) of "Heloxy 8" (manufactured by ACI Japan Ltd.) over a period of 3 hours, and the mixture was stirred for further 3 hours. The reaction mixture, from which the absence of the glycidyl ether had been confirmed by TLC (thin layer chromatography) and gas chromatography, was cooled to 40°C or lower, and added with 18.6 g (0.18 mols) of 35 % HCl. The mixture was adjusted in pH with additional 35 % HCl to 5.2, whereby 209 g of a liquid composition having a solid content of about 60.0 % was obtained. Production Example 2:

### N-(2-hydroxy-3-dodecyloxy)propyl-L-arginine hydrochloride

17.4 g (0.1 mols) of L-arginine was dissolved in 100 ml of water in a three-neck, round-bottomed flask, to which was added 100 ml of isopropanol. While the mixture was heated and stirred under reflux, 24.2 g (0.1 mols) of dodecylglycidyl ether (manufactured by Sakamoto Yakuhin Kogyo Co., Ltd.) was dropwise added thereto over a period of 30 minutes, and was further stirred still under reflux for 3 hours. The reaction mixture, from which the absence of the dodecylglycidyl ether had been confirmed by TLC and gas chromatography, was cooled to 40°C or lower and then neutralized with 10.1 g (0.1 mols) of 36 % HCl added thereto.

The mixture was concentrated under reduced pressure, and the resulting residue was purified through silica gel column chromatography (manufactured by Merck & Co., Inc.; Kieselgel 60 using a developer of chloroform/methanol/acetic acid = 3/1/0.5) to obtain 15.0 g (yield: 36.0 %) of N-(2-hydroxy-3-dodecyloxy)propyl-L-arginine hydrochloride.
TLC (butanol/acetic acid/water = 4/1/2):Rf = 0.64.
ESI (Electrospray Ionization) Mass Spectrum:417.5 (MH⁺).
IR (NaCl, cm⁻¹):3177, 2955, 2920, 2853, 1962, 1628, 1468, 1397, 1377, 1215, 1116.

### Production Example 3:

### N,N-bis(2-hydroxy-3-dodecyloxy)propyl-L-arginine hydrochloride

17.4 g (0.1 mols) of L-arginine was dissolved in 100 ml of water in a three-neck, round-bottomed flask, to which was added 100 ml of isopropanol. While the mixture was heated and stirred under reflux, 28.4 g (0.2 mols) of dodecylglycidyl ether was dropwise added thereto over a period of 30 minutes, and was further stirred still under reflux for 3 hours. The reaction mixture, from which the absence of the dodecylglycidyl ether had been confirmed by TLC and gas chromatography, was cooled to 40°C or lower and then neutralized with 10.1 g (0.1 mols) of 36 % HCl.

The mixture was concentrated under reduced pressure, and the resulting residue was purified through silica gel column chromatography (Kieselgel 60 using a developer of chloroform/methanol/acetic acid = 3/1/0.5) to obtain 11.4 g (yield: 17.2 %) of N,N-bis(2-hydroxy-3-dodecyloxy)propyl-L-arginine hydrochloride.
TLC (butanol/acetic acid/water = 4/1/2):Rf = 0.72.
ESI Mass Spectrum:659.7 (MH⁺).
IR (NaCl, cm⁻¹):3177, 2955, 2920, 2853, 1692, 1628, 1468, 1397, 1377, 1215, 1120.

### Production Example 4:

### N-(2-hydroxy-3-octadecyloxy)propyl-L-arginine hydrochloride

17.4 g (0.1 mols) of L-arginine and 32.6 g (0.1 mols) of octadecylglycidyl ether (manufactured by Sakamoto Yakuhin Kogyo Co., Ltd.) were used in the same manner as in Production Example 2 to obtain 21.2 g (yield: 42.3 %) of N-(2-hydroxy-3-octadecyloxy)propyl-L-arginine hydrochloride.
TLC (butanol/acetic acid/water = 4/1/2):Rf = 0.64.
ESI Mass Spectrum:501.5 (MH⁺).
IR (NaCl, cm⁻¹):3175, 2955, 2917, 2851, 1692, 1628, 1468, 1377, 1215, 1121.

### Production Example 5:

### Nε-(2-hydroxy-3-dodecyloxy)propyl-L-lysine hydrochloride

18.3 g (0.1 mols) of L-lysine hydrochloride and 8.0 g (0.2 mols) of sodium hydroxide were dissolved in 100 ml of water in a three-neck, round-bottomed flask, to which was added 100 ml of isopropanol. While the mixture was heated and stirred under reflux, 24.2 g (0.1 mols) of dodecylglycidyl ether was dropwise added thereto over a period of 30 minutes, and was further stirred still under reflux for 3 hours. The reaction mixture, from which the absence of the dodecylglycidyl ether had been confirmed by TLC and gas chromatography, was cooled to 40°C or lower and then neutralized with 10.1 g (0.1 mols) of 36 % HCl.

The mixture was concentrated under reduced pressure, and the resulting residue was purified through silica gel column chromatography (Kieselgel 60 using a developer of chloroform/methanol/acetic acid = 3/1/0.5) to obtain 10.1 g (yield: 23.7 %) of Nε-(2-hydroxy-3-dodecyloxy)propyl-L-lysina hydrochloride.
TLC (butanol/acetic acid/water = 4/1/2):Rf = 0.42.
ESI (Electrospray Ionization) Mass Spectrum:389.4 (MH⁺).
IR (NaCl, cm⁻¹):2955, 2923, 2853, 1620, 1586, 1468, 1120.

### Production Example 6:

17.4 g (0.1 mols) of L-arginine was dissolved in 100 ml of water in a three-neck, round-bottomed flask, to which was added 100 ml of isopropanol. While the mixture was heated and stirred under reflux, 25.6 g (0.1 mols) of "Holoxy 8" (manufactured by ACI Japan Ltd.) was dropwise added thereto over a period of 30 minutes, and was further stirred still under reflux for 3 hours. The reaction mixture, from which the absence of the glycidyl ethers had been confirmed by TLC and gas chromatography, was cooled to 40°C or lower and then added with 12.9 g (0.1 mols) of DL-pyrrolidonecarboxylic acid.

The mixture was concentrated by evaporating the isopropanol under reduced pressure, and the resulting residue was poured into acetone. The resulting crystals were separated and dried to obtain 50.9 g of a mixture of N-(2-hydroxy-3-dodecyloxy)propyl-L-arginine DL-pyrrolidonecarboxylic acid salt and N-(2-hydroxy-3-tetradecyloxy)propyl-L-arginine DL-pyrrolidonecarboxylic acid salt.
ESI Mass Spectrum:417.5 (MH⁺), 445.5 (MH⁺).

### Production Example 7:

### N-(2-hydroxydodecyl)-L-arginine hydrochloride

17.4 g (0.1 mols) of L-arginine and 18.4 g (0.1 mols) of 1.2 - epoxydodecane were used in the same manner as in Production Example 2 to obtain 13.0 g (yield: 32.9 %) of N-(2-hydroxydodecyl)-L-arginine hydrochloride.

### Test Example I (Comparative Examples 1 to 4 and Examples 1 to 10):

Hair rinse samples each having a composition shown in Table 1 below (in which all the components are represented in terms of % by weight relative to the total weight of the composition, 100 %) were prepared. After having shampooed, a panel of five specialists tried those rinse samples, and sensually evaluated their hair with respect to (a) the sticky feel while the hair was wet with the rinse, (b) the sticky feel after the hair was dried, (c) the moist feel of the hair, (d) the smooth combability, and (e) the sleekness of the hair. For (c), (d) and (e), their hair after dried was evaluated. Regarding the product of Production Example 1, it was previously freeze-dried to remove water and ethanol, and then formulated into rinse samples. The test results are also shown in Table 1.

In Table 1, each sample was evaluated relative to the standard sample of Comparative Example 1. The points that each sample gained according to the evaluation criteria mentioned below were averaged. The sample having an average point of 4.5 or higher is excellent (A); that having an average point of from 3.5 to 4.4 is good (B); that having an average point of from 2.5 to 3.4 is acceptable (C); and that having an average point of 2.4 or lower is bad (D).

### <Evaluation Criteria>

(a) Sticky feel while wet:
   - 5:: Less sticky than the standard.
   - 4:: Somewhat less sticky than the standard.
   - 3:: Same as the standard.
   - 2:: Somewhat more sticky than the standard.
   - 1:: More sticky than the standard.
(b) Sticky feel after dried:
   - 5:: Less sticky than the standard.
   - 4:: Somewhat less sticky than the standard.
   - 3:: Same as the standard.
   - 2:: Somewhat more sticky than the standard.
   - 1:: More sticky than the standard.
(c) Moist feel of the hair:
   - 5:: More moist than the standard.
   - 4:: Somewhat more moist than the standard.
   - 3:: Same as the standard.
   - 2:: Somewhat less moist than the standard.
   - 1:: Less moist than the standard.
(d) Smooth combability:
   - 5:: Smoother than the standard.
   - 4:: Somewhat smoother than the standard.
   - 3:: Same as the standard.
   - 2:: Somewhat less smooth than the standard.
   - 1:: Less smooth than the standard.
(e) Sleekness of the hair:
   - 5:: Sleeker than the standard.
   - 4:: Somewhat sleeker than the standard.
   - 3:: Same as the standard.
   - 2:: Somewhat less sleek than the standard.
   - 1:: Less sleek than the standard.

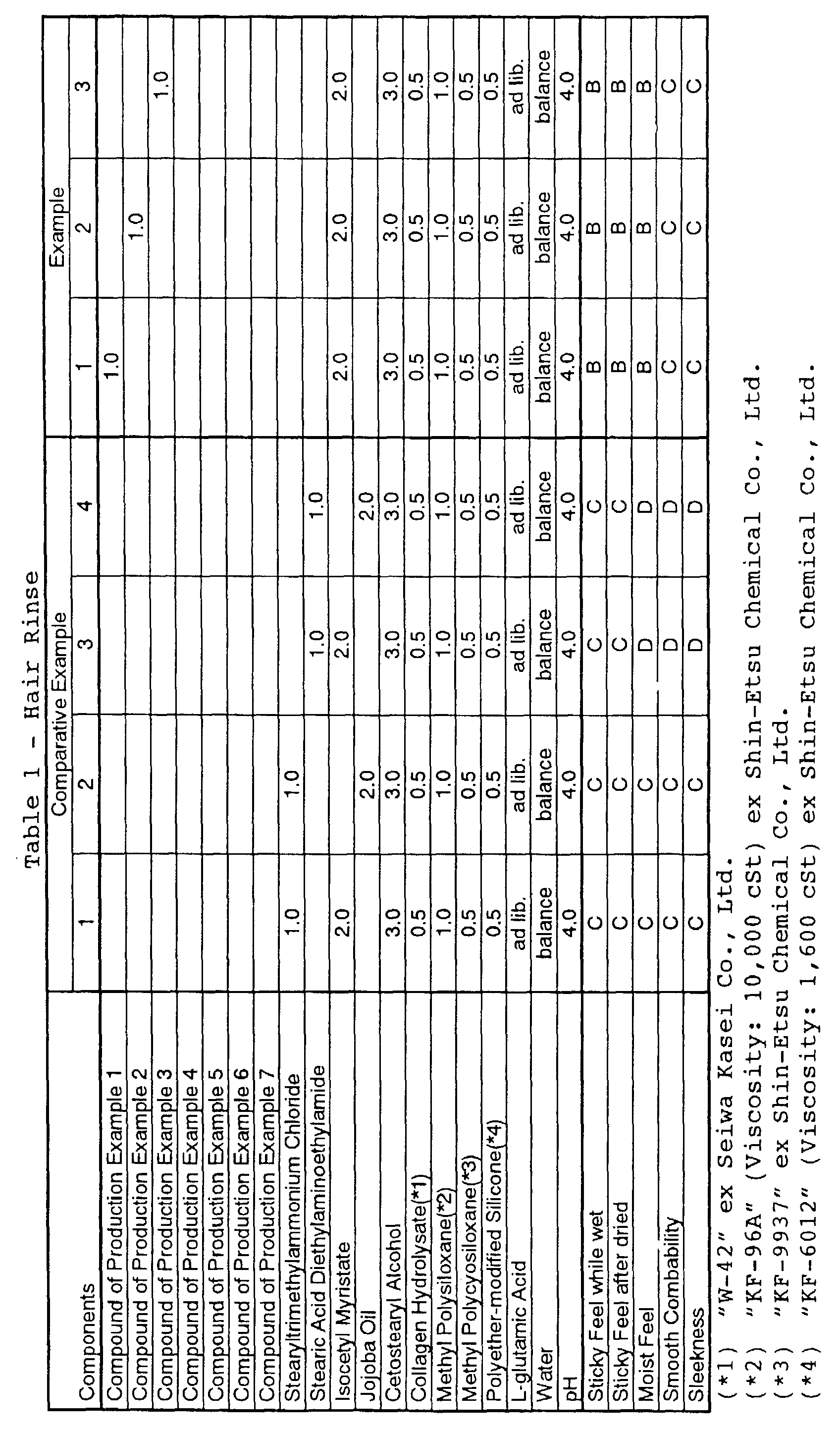

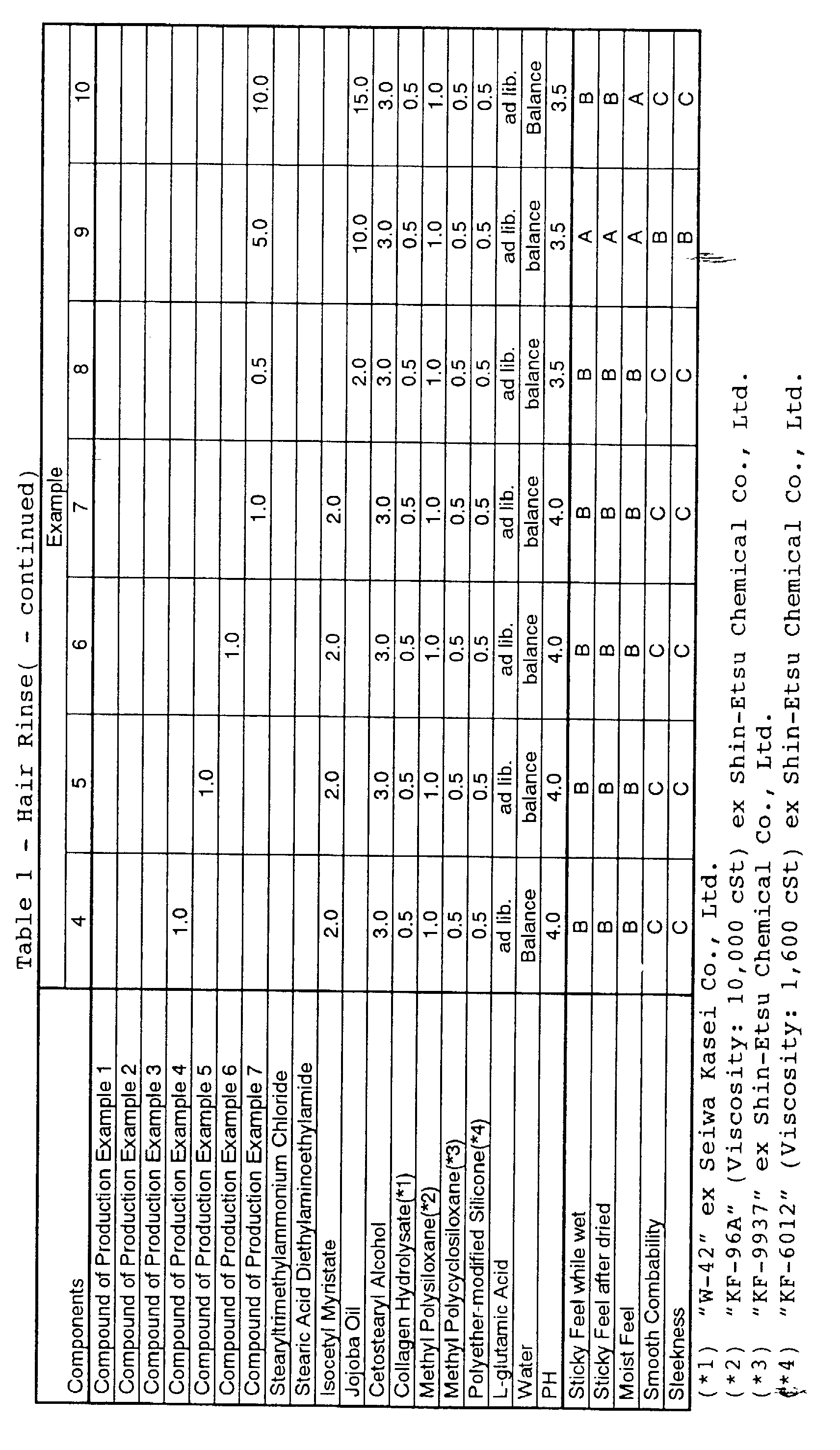

### Test Example II (Comparative Examples 5 to 8, and Examples 11 to 13):

Hair cream samples each having a composition shown in Table 2 below (in which all the components are represented in terms of % by weight relative to the total weight of the composition, 100 %) were prepared. These were each separately applied to hair wicks (natural human hair models on the head of mannequins), and sensually evaluated by a panel of five specialists with respect to (a) the sticky feel while the hair was wet with the hair cream, (b) the sticky feel after the hair was dried, (c) the moist feel of the hair, (d) the smooth combability, and (e) the sleekness of the hair. For (c), (d) and (e), the hair wicks after dried were evaluated. In Table 2, each sample was evaluated relative to the standard sample of Comparative Example 5, in the same manner as in Test Example I. The test results are also shown in Table 2.

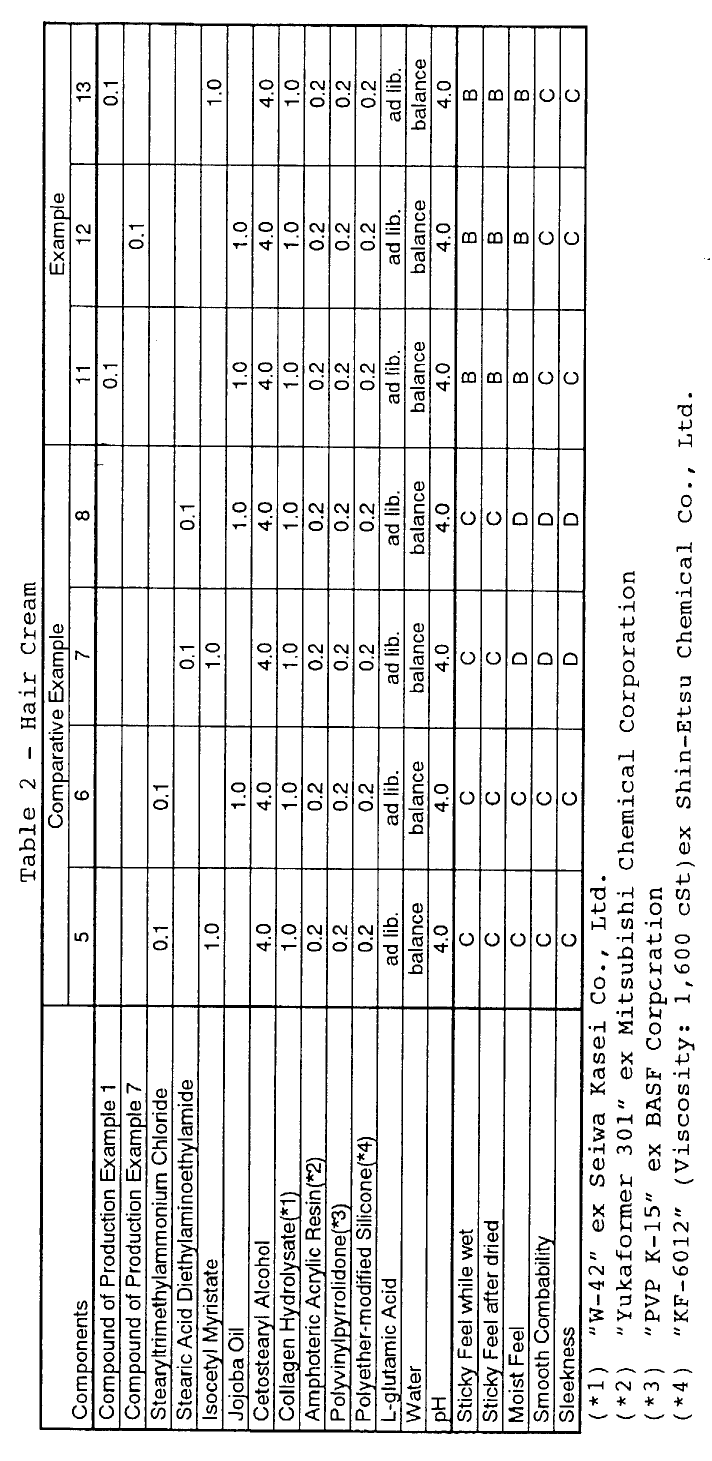

### Test Example III (Comparative Examples 9 and 10, and Examples 14 and 15):

Hair shampoo samples each having a composition shown in Table 3 below (in which all the components are represented in terms of % by weight relative to the total weight of the composition, 100 %) were prepared. Hair pieces were shampooed with any of those samples, dried, and sensually evaluated by a panel of five specialists with respect to (c) the moist feel of the hair, (d) the smooth combability, and (e) the sleekness of the hair. In Table 3, each sample was evaluated relative to the standard sample of Comparative Example 9, in the same manner as in Test Example I. The test results are also shown in Table 3.

**Table 3 -**

| Hair Shampoo | | | | |
|---|---|---|---|---|
| | Comparative Example | | Example | |
| Components | 9 | 10 | 14 | 15 |
| Compound of Production Example 1 | | | 1 | |
| Compound of Production Example 7 | | | | 1 |
| Stearytrimethylammonium Chloride | 1 | 1 | | |
| Sodium Laurylether Sulfate(*1) | 10 | 10 | 10 | 10 |
| Amino Acid-based Oily Material(*2) | 0.5 | | 0.5 | 1 |
| Polyoxyethylene(40) hardened Castor Oil Pyroglutamic Acid Isostearic Acid Disester | 1 | 1 | 1 | 1 |
| Coconut Oil Fatty Acid Diethanolamide | 3 | 3 | 3 | 3 |
| Cationized Cellulose(*3) | 0.3 | 0.3 | 0.3 | 0.3 |
| Glycerine | 2 | 2 | 2 | 2 |
| Polyether-modified Silicone(*4) | 1 | 1 | 1 | 1 |
| Water | balance | balance | balance | balance |
| Moist Feel | C | D | B | B |
| Smooth Combability | C | C | C | C |
| Sleekness | C | C | C | C |

| | | | | |
|---|---|---|---|---|
| (*1) "Emal 20C" ex Kao Corporation | | | | |
| (*2) "Eldew CL-203" ex Ajinomoto Co., Inc. | | | | |
| (*3) "Leogard GP" ex Lion Corporation | | | | |
| (*4) "KF-9937" ex Shin-Etsu Chemical Co., Ltd. | | | | |

### Test Example IV (Comparative Examples 11 and 12, and Examples 16 to 19):

Milky lotion samples each having a composition shown in Table 4 below (in which all the components are represented in terms of % by weight relative to the total weight of the composition, 100 %) were prepared. A panel of five panelists tried and sensually evaluated them on the backs of their hands, with respect to (a) the sticky feel, (b) the affinity for the skin, and (c) the moist feel.

In Table 4, each sample was evaluated relative to the standard sample of Comparative Example 11. The points that each sample gained according to the evaluation criteria mentioned below were averaged. The sample having an average point of 4.5 or higher is excellent (A); that having an average point of from 3.5 to 4.4 is good (B); that having an average point of from 2.5 to 3.4 is acceptable (C); and that having an average point of 2.4 or lower is bad (D). The results are also in Table 4.

### <Evaluation Criteria>

(a) Sticky feel:
   - 5:: Less sticky than the standard.
   - 4:: Somewhat less sticky than the standard.
   - 3:: Same as the standard.
   - 2:: Somewhat more sticky than the standard.
   - 1:: More sticky than the standard.
(b) Affinity:
   - 5:: Better than the standard.
   - 4:: Somewhat better than the standard.
   - 3:: Same as the standard.
   - 2:: Somewhat poorer than the standard.
   - 1:: Poorer than the standard.
(c) Moist feel:
   - 5:: More moist than the standard.
   - 4:: Somewhat more moist than the standard.
   - 3:: Same as the standard.
   - 2:: Somewhat less moist than the standard.
   - 1:: Less moist than the standard.

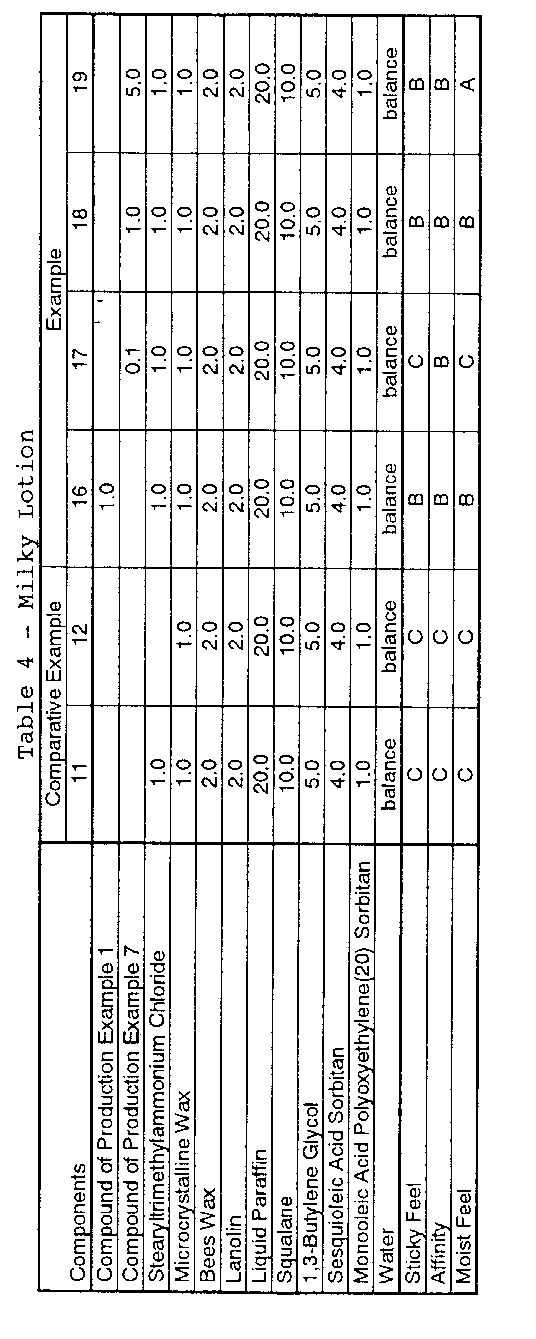

### Reference Example 1 (Safety):

The primary irritation of the skin and ocular mucous membranes by some test compounds mentioned below was checked according to the test methods mentioned below.

### (1) Test for primary irritation of the skin:

A patch test plaster, into which 0.3 ml of an aqueous 1 % solution of a test compound had been infiltrated, was airtightly stuck on each of four male New Zealand white rabbits and kept as such for 24 hours. After the plaster was removed, the rabbits were observed for 24 hours as to whether or not their skin was irritated by the compound, according to the Draize's skin irritation standard. The compounds tested were evaluated on the basis of the following criteria.

| <Evaluation Criteria> | |
|---|---|
| Draize Point | Irritation |
| 4 or more | Heavy |
| 2 to less than 4 | Light |
| Less than 2 | Minor |

### (2) Test for primary irritation of ocular mucous membranes:

The lower eyelids of each of four male New Zealand white rabbits were formed into bags, into which was dropped 0.1 ml of an aqueous surfactant solution, and the upper and lower eyelids were lightly ligated. 24 hours after the dropping, the irritation of the ocular mucous membranes of each rabbit was checked according to the Draize's points mentioned below. The compound tested was evaluated on the basis of the following criteria.

| <Evaluation Criteria> | |
|---|---|
| Draize Point | Irritation |
| 50 or more | Heavy |
| 20 to less than 50 | Moderate |
| 10 to less than 20 | Light |
| Less than 10 | Minor |

Five compounds shown in Table 5 below were tested for (1) and (2) noted above. The results are also shown in the same Table.

**Table 5:**

| Safety | | |
|---|---|---|
| Compounds | Primary Skin Irritation | Primary Mucous Membrane Irritation |
| Compound of Production Example 5 | Minor | Minor |
| Compound of Production Example 6 | Minor | Minor |
| Compound of Production Example 7 | Minor | Minor |
| Stearyltrimethylammonium Chloride | Heavy | Moderate |
| Cocoylarginine ethyl ester DL-pyrrolidonecarboxylic Acid Salt | Minor | Minor |

### Reference Example 2 (Biodegradability Test):

Herein demonstrated is a biodegradability test as carried out for 28 days according to OECD Chemicals Test Guideline 301C Modified MITI Test (I) - 1981. In this test was used activated sludge as collected from a sewage treatment plant, as the microorganisms source, and its biological oxygen demand (BOD) was continuously measured using a closed-system, biological oxygen demand-measuring device (BOD-measuring device).

Precisely, a test compound (compound of Production Example 6) was subjected to a BOD-based biodegradability test according to the test design shown in Table 6 below. The test data obtained are shown in Table 6, from which it is known that the biodegradability of the test compound for 28 days was 60.0 %. In the control section, the biodegradability of aniline for 7 days was higher than 40 %, which validates the present test. On the other hand, in the comparative section, the biodegradability of the comparative compound, stearyltrimethylammonium chloride (one of monoalkyltrimethylammonium chlorides) for 28 days was 0.0 %, which indicates that the comparative compound is not biodegradable.

**Table 6 :**

| Biodegradability Test Data (%) | | | | |
|---|---|---|---|---|
| Test Group | 7 days | 14 days | 21 days | 28 days |
| Compound of Production Example 6 | | | | |
| Cultivation Test 1 | 16.1 | 40.4 | 52.2 | 55.5 |
| Cultivation Test 2 | 18.4 | 38.1 | 57.2 | 67.3 |
| Cultivation Test 3 | 16.1 | 42.6 | 55.6 | 57.2 |
| Average | 16.9 | 40.4 | 55.0 | 60.0 |

| Stearyltrimethylammonium Chloride | | | | |
|---|---|---|---|---|
| Cultivation Test 1 | 0.0 | 0.0 | 0.0 | 0.0 |
| Cultivation Test 2 | 0.0 | 0.0 | 0.0 | 0.0 |
| Cultivation Test 3 | 0.0 | 0.0 | 0.0 | 0.0 |
| Average | 0.0 | 0.0 | 0.0 | 0.0 |

| Aniline | | | | |
|---|---|---|---|---|
| Control Test | 65.4 | 72.4 | 75.7 | 77.7 |

According to the present invention, a cosmetic composition, which exhibits, when applied to the hair, an excellent hair-conditioning effect for smooth combing, sleeking and wet feeling with no stickiness, and exhibits, when applied to the skin, good affinity for, and good moisturizing effect on, the skin with no stickiness can be easily obtained.

## Claims

1. A cosmetic composition comprising, as the active ingredient, at least one member selected from the group consisting of basic amino acid derivatives of the following general formula (1) and their salts (Component A) and at least one member selected from the group consisting of natural, mineral and synthetic oily materials for cosmetic use (Component B), 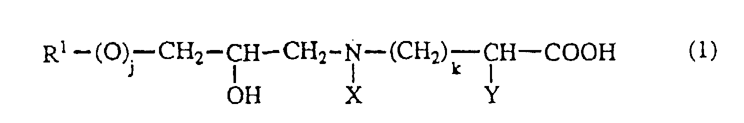 Wherein in Formula (1), R¹ represents a straight-chain or branched-chain alkyl or alkenyl group having from 8 to 22 carbon atoms; j represents an integer of 0 or 1; X represents a hydrogen atom or a substituent of the following general formula (2); k represents an integer of from 0 to 5; and Y represents a substituent of the following general formula (3) when k = 0, while Y represents an amino group when k = an integer of from 1 to 5, 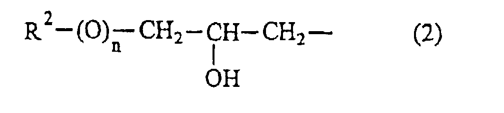 Wherein Formula (2), R² represents a straight-chain or branched-chain alkyl or alkenyl group having from 8 to 22 carbon atoms, which may be the same as, or different from, R¹; and n represents an integer of 0 or 1, 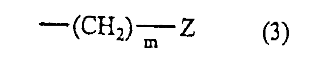 and wherein in Formula (3), m represents an integer of from 1 to 5;
and Z represents a substituent of any of the following (I) to (IV).
